(19) Europäisches Patentamt
European Patent Office
Office européen des brevets

(11) **EP 3 438 268 B1**

(12) **EUROPÄISCHE PATENTSCHRIFT**

(45) Veröffentlichungstag und Bekanntmachung des Hinweises auf die Patenterteilung:
**01.04.2020 Patentblatt 2020/14**

(51) Int Cl.:
*C12P 5/02* (2006.01)     *B01J 23/745* (2006.01)
*C10G 45/12* (2006.01)

(21) Anmeldenummer: **17184091.1**

(22) Anmeldetag: **31.07.2017**

(54) **VERWENDUNG VON EISENHYDROXID BEI DER BIOGASERZEUGUNG ALS SCHWEFELWASSERSTOFF AUSFÄLLENDER ZUSATZ ZUR BIOMASSE**

USE OF IRON HYDROXIDE IN BIOGAS PRODUCTION AS HYDROGEN SULPHIDE ADDITIVE FOR BIOMASS

UTILISATION D'HYDROXYDE FERRIQUE LORS DE LA PRODUCTION DE BIOGAZ EN TANT QU'ADDITIF PRÉCIPITANT D'HYDROGÈNE SULFURÉ À LA BIOMASSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.02.2019 Patentblatt 2019/06**

(73) Patentinhaber: **ISF GmbH**
**23812 Wahlstedt (DE)**

(72) Erfinder:
• **KURZBUCH, Marc**
**22869 Schenefeld (DE)**
• **MATHIES, Edmund**
**25436 Moorrege (DE)**
• **RAMHOLD, Dietmar**
**23820 Pronstorf (DE)**

(74) Vertreter: **Hauck Patentanwaltspartnerschaft mbB**
**Postfach 11 31 53**
**20431 Hamburg (DE)**

(56) Entgegenhaltungen:
**EP-A2- 2 998 386**     **DE-A1-102007 003 532**

• **DATABASE WPI Week 200227 Thomson Scientific, London, GB; AN 2002-210739 XP002774921, -& JP 2001 316302 A (JAPAN STEEL WORKS LTD) 13. November 2001 (2001-11-13)**
• **DATABASE WPI Week 201654 Thomson Scientific, London, GB; AN 2016-35750C XP002774922, -& CN 105 617 996 A (WUXI XINENG BOILER CO LTD) 1. Juni 2016 (2016-06-01)**
• **DATABASE WPI Week 200872 Thomson Scientific, London, GB; AN 2008-M28496 XP002774923, & KR 2008 0045818 A (SAMCHULLY CO LTD) 26. Mai 2008 (2008-05-26)**

EP 3 438 268 B1

## Beschreibung

Die Erfindung betrifft die Verwendung von Eisenhydroxid bei der Biogaserzeugung als Schwefelwasserstoff ausfällender Zusatz zur Biomasse, wobei zusätzlich Zeolith als die Reaktionsgeschwindigkeit der Ausfällung des Schwefelwasserstoffs durch Eisenhydroxid erhöhender Zusatz zur Biomasse verwendet wird.

Biogas ist ein brennbares Gasgemisch, das beim anaeroben Abbau von organischen Stoffen (Mist, Grünpflanzen, Klärschlamm, Biomüll etc.) insbesondere durch Methanbakterien gebildet wird. Biogas besteht durchschnittlich aus 50 bis 75 Volumenprozent Methan, 20 bis 45 Volumenprozent Kohlendioxid, 2 bis 7 Volumenprozent Wasser, 20 bis 20.000 ppm Schwefelwasserstoff, jeweils unter 2 Volumenprozent Stickstoff und Sauerstoff und unter 1 Volumenprozent Wasserstoff.

Der Prozess der Biogaserzeugung kann in vier Stufen unterteilt werden. In einem ersten Schritt, der Hydrolyse, werden die komplexen Strukturen der Biomasse in ihre Monomere (Zucker, Fette, Eiweiße) zerlegt. Anschließend erfolgt ein Abbau der Monomere zu kurzkettigen Fettsäuren (Acidogenese). Im dritten Schritt (Acetogenese) und im vierten Schritt (Methanogenese) erfolgt zunächst die Bildung von Essigsäure und in der Folge von Methan. Zudem fallen obige Nebenprodukte an. Der Schwefelwasserstoff entsteht bei anaeroben Abbauprozessen von organischen und anorganischen Schwefelverbindungen. Die optimalen Milieubedingungen unterscheiden sich in den jeweiligen Schritten zum Teil erheblich (Sahm: Biologie der Methanbildung, Chem.-Ing. Tech. 53 (1981) Nr. 11, Seiten 854-863).

In der Regel erfolgt der anaerobe Abbau organischer Substanz im wässrigen Milieu mit Trockensubstanzgehalten von 3 bis 30 %, vorzugsweise von 5 bis 15 %.

Die Erzeugung von Biogas erfolgt in Abhängigkeit der am Prozess beteiligten Mikroorganismen bei verschiedenen Temperaturoptima im Bereich von 25 bis 60 C.

Biogas wird insbesondere für die Energiegewinnung in Blockheizkraftwerken (BHKW) verwendet, um elektrische Energie und Wärme zu erzeugen. Schwefelwasserstoff und seine bei der Verbrennung im BHKW auftretenden Reaktionsprodukte (Schweflige Säure, Schwefelsäure und andere) können aufgrund ihrer stark korrosiven Wirkung die Standzeiten von Blockheizkraftwerken erheblich herabsetzen. Zudem kann Schwefelwasserstoff Oxidationskatalysatoren von BHKW-Motoren vergiften. Nachteilig ist ferner die toxische Wirkung von im Wasser gelösten Schwefelwasserstoff auf die methanogenen Mikroorganismen. Zudem werden durch Schwefelwasserstoff von Methanbildnern benötigte Spurenelemente ausgefällt. Deshalb wird angestrebt, Biogas mit möglichst geringen Anteilen Schwefelwasserstoff zu erzeugen und ebenso die Schwefelwasserstoff Gehalte im Gärsubstrat zu verringern. Eine Möglichkeit zur wirksamen Reduzierung des Schwefelwasserstoffgehaltes ist die Zugabe von Eisenhydroxid (Eisen(III)-Oxihydrat) zum flüssigen Gärsubstrat. Hierdurch wird Schwefelwasserstoff unter Bildung von schwarzem feindispersem Eisensulfid ausgefällt, das mit dem Gärrest aus dem Bioreaktor ausgetragen wird (Dr. Andreas Otto, Dipl.-Ing. Detlef Güßbacher, Dipl.-Ing. agr. Jenny Conrad "Möglichkeiten des Einsatzes von Eisenhydroxid für die Bindung von Schwefelwasserstoff in Anaerob-Prozessen").

Die Entschwefelungsleistung des Eisenhydroxid hängt vom Verhältnis der amorphen und kristallinen Strukturen im Eisenhydroxid ab. Je höher der kristalline Anteil, desto schneller ist die Reaktion mit Sulfid bzw. Schwefelwasserstoff (Dr. Sabine Rahn, Detlef Güßbacher, Evgenij Marcenko "Eisenhydroxid ist nicht gleich Eisenhydroxid", Joule 5.20154, Seiten 58 bis 60).

Die JP 60 014 995 A beschreibt ein Verfahren zur Verbesserung der Fermentation bei der Erzeugung von Methangas. Hierfür werden dem Fermentationsprozess natürliche Feststoffe beigemengt, wobei neben zahlreichen Silikatmaterialien auch das Mineral Zeolith verwendet wird. Gemäß EP 1 577 269 B2 wird mehrfach modifizierter Zeolith als prozessstabilisierender, den Abbau von Gärsubstrat aktivierender und in Biogas vorhandenes Ammoniak- und Schwefelwasserstoff senkender Zusatz sowie als zellteilungsoptimierender und faulzeitverkürzender Zusatz eines Gärsubstrats in einer Menge von etwa 0,1 % bis zu etwa 10 % bezogen auf die organische Substanz des Gärsubstrats verwendet. Dabei wird unter einem modifizierten Zeolith, der auch als aktivierter Zeolith bezeichnet wird, ein chemisch und/oder chemisch-physikalisch behandelter Zeolith verstanden, wobei nach Modifizierung ein veränderter Zeolith vorliegt. Unter chemisch, chemisch-physikalisch behandelter Zeolith fällt auch ein beladener, versetzter Zeolith. Dementsprechend weist ein modifizierter Zeolith Fremdionen und/oder andere Fremdstoffe auf.

Die DE 10 2007 003 532 A1 beschreibt einen geträgerten Metallkatalysator für den Einsatz als Oxidationskatalysator schwefelhaltige Abgase, der weitgehend beständig gegenüber Schwefelverbindungen ist. Der Metallkatalysator umfasst ein Zeolithmaterial, dessen innere Oberfläche mit Metallpartikeln und dessen äußere Oberfläche mit einem Eisenoxid beladen ist.

Die JP 2001316302 A beschreibt einen pulverförmigen Katalysator, der Eisenoxid und Zeolith umfassen kann, zur Entfernung von Schwefelwasserstoff aus Biogas.

Die CN 105 617 996 A beschreibt die Entschwefelung von Abgasen aus einem Biomassekessel mithilfe eines Entschwefelungsmittels, das eine Mischung aus unterschiedlichen Komponenten ist. Zu den Komponenten gehören Kalkstein, Eisenoxid, Mordenit, Aluminiumchlorid, Dimethylformamid und Ammoniakwasser.

Die KR 20080045818 A beschreibt ebenfalls die Entschwefelung von Gasen wie Brenngas oder Erdgas durch Absorption einer Mischung aus Aktivkohle oder Aktivkohle und Zeolith mit einem oder mehreren weiteren Stoffen, zu denen u.a. Eisenoxid gehört.

Die EP 298 386 A2 beschreibt ein Verfahren zur Biogaserzeugung, bei dem der Biomasse ein die Biogasproduktion unterstützendes Stoffgemisch (Biogas-Booster) beigefügt wird. Der Biogas-Booster enthält aquatische Pflanzen mit Beimengungen von Fettsäuren und/oder Fettsäureestern. Zur Adaption des Biogas-Boosters an individuelle Anlagen sind diesem zusätzlich Eisenhydroxid und/oder pH-Regulator und/oder Zeolithe und/oder verseifte Rückstände aus der Lebensmittel- oder Speiseölproduktion und/oder Enzyme und/oder Mineralien und/oder gemahlene Aktiv- und/oder Holzkohle zugefügt.

Davon ausgehend liegt der Erfindung die Aufgabe zugrunde, ein Verfahren zur Biogaserzeugung unter wirksamerer Verringerung der Schwefelwasserstoffkonzentration in Biogas zu schaffen.

Die Aufgabe wird erfindungsgemäß durch eine Verwendung gemäß Anspruch 1 gelöst. Vorteilhafte Ausführungsarten der Erfindung sind in Unteransprüchen angegeben.

Erfindungsgemäß ist die Verwendung von Eisenhydroxid bei der Biogaserzeugung als Schwefelwasserstoff ausfällender Zusatz zur Biomasse dadurch gekennzeichnet, dass zusätzlich Zeolith als die Reaktionsgeschwindigkeit der Ausfällung des Schwefelwasserstoffs durch Eisenhydroxid erhöhender Zusatz zur Biomasse verwendet wird.

Gemäß der Erfindung wird durch die kombinierte Zugabe des Eisenhydroxids und des Zeoliths zur Biomasse die Reaktionsgeschwindigkeit der Ausfällung des Schwefelwasserstoffs aus der Flüssigkeit in der Biomasse (flüssiges Gärsubstrat) bei der Biogaserzeugung erhöht. Hierdurch wird die Konzentration des Schwefelwasserstoffs im flüssigen Gärsubstrat verringert. Damit wird auch der Schwefelwasserstoff im Biogas verringert, der mit dem Schwefelwasserstoff in der Flüssigphase im Gleichgewicht steht. Die schädlichen Einflüsse des Schwefelwasserstoffs auf die Biologie der Biogaserzeugung und auf die der Biogaserzeugung nachgeschaltete Anlagentechnik werden hierdurch herabgesetzt. Die Erhöhung der Reaktionsgeschwindigkeit der Ausfällung des Schwefelwasserstoffs durch Zugabe von Zeolith war bislang nicht bekannt.

Durch die erfindungsgemäße Verwendung von Eisenhydroxid und Zeolith kann sowohl die Zeit für den Beginn (Reaktionsbeginn) als auch die Zeit für die Dauer (Reaktionsdauer) der Umsetzung des Eisenhydroxids mit Schwefelwasserstoff zu Eisensulfid verringert werden, das in der flüssigen Phase des Gärsubstrats ausfällt. Somit bezeichnet die Reaktionsgeschwindigkeit mindestens einen der Parameter Reaktionsbeginn und Reaktionsdauer. Gemäß einer bevorzugten Ausführungsart bezeichnet die Reaktionsgeschwindigkeit den Reaktionsbeginn und die Reaktionsdauer.

Gemäß einer Ausführungsart der Erfindung werden das Eisenhydroxid und der Zeolith vorgemischt und der Biomasse während der Biogaserzeugung zugeführt. Während der Biogaserzeugung befindet sich die Biomasse im Biogasreaktor (Fermenter) und wird auch als "Gärsubstrat" bezeichnet. Hierdurch kann sichergestellt werden, dass Eisenhydroxid und Zeolith bei der Biogaserzeugung in der die Reaktionsgeschwindigkeit erhöhenden Kombination zur Verfügung steht. Gemäß einer anderen Ausführungsart werden das Eisenhydroxid und der Zeolith der Biomasse während der Biogaserzeugung gesondert zugeführt, so dass sich in der Biomasse die vorteilhafte Kombination von Eisenhydroxid und Zeolith einstellt. Gemäß einer bevorzugten Ausführungsart werden Eisenhydroxid und Zeolith während der Biogaserzeugung in die Biomasse eingemischt. Hierdurch wird erreicht, dass die Kombination aus Eisenhydroxid und Zeolith bei der Biogaserzeugung überall in der Biomasse vorliegt.

Gemäß einer weiteren Ausführungsart werden das Eisenhydroxid und der Zeolith gemeinsam mit der Biomasse (Fütterung) dem Biogasreaktor zugeführt. Gemäß einer weiteren Ausführungsart werden das Eisenhydroxid und der Zeolith mit der zu fermentierenden Biomasse vorgemischt und die Vormischung dem Gärprozess zugeführt. Bei dieser Ausführungsart steht die Kombination aus Eisenhydroxid und Zeolith bereits zu Beginn des Vergärungsprozesses zur Verfügung.

Gemäß einer weiteren Ausführungsart werden das Eisenhydroxid und der Zeolith mittels Feststoffschnecken der Biomasse in einem Biogasreaktor zugeführt. Mittels Feststoffschnecken kann die Zugabe von Eisenhydroxid und Zeolith dosiert werden. Ferner kann mittels der Feststoffschnecken die Vermischung von Eisenhydroxid und Zeolith und gegebenenfalls der zu fermentierenden Biomasse erfolgen oder fortgesetzt werden.

Gemäß einer weiteren Ausführungsart wird der Biomasse ein pulverförmiges Reaktionsmittel auf der Basis von Eisenhydroxid mit einem Eisengehalt von mindestens 10 %, vorzugsweise von mindestens 20 %, vorzugsweise von mindestens 33 % bezogen auf den Trockenstoff und einem Trockenstoffgehalt von mindestens 50 %, vorzugsweise von mindestens 70 %, vorzugsweise von mindestens 85 % zugeführt.

Das pulverförmige Reaktionsmittel kann zusätzlich zu Eisenhydroxid Manganverbindungen und weitere Beimengungen wie Mengen- und Spurenelementeverbindungen enthalten. Für die Verwendung als pulverförmiges Reaktionsmittel geeignet sind unter der Produktbezeichnung FerroSorp ® DGp und FerroSorp ® DGp+K von der Firma HeGo Biotec GmbH, Goerzallee 305 b, 14167 Berlin, Deutschland vermarktete Produkte oder ähnliche.

Die vorbezeichneten Produkte weisen die in den Tabellen am Ende der Figurenbeschreibung angegebenen Eigenschaften auf.

Gemäß einer weiteren Ausführungsart wird mindestens ein natürlicher Zeolith verwendet. Hierbei handelt es sich um einen unbehandelten Zeolith. Bevorzugt wird einer oder werden Mischungen mehrerer der nachfolgenden Zeolithe verwendet: Klinoptilolith, Chabasit, Phillipsit, Analcim oder ähnliche.

Als Zeolith kann ein Klinoptilolith sedimentären Ursprungs (E 568) verwendet werden. Als Zeolith kann weiterhin ein aufbereitetes Naturgestein verwendet werden, dessen wesentlicher Bestandteil hydratisiertes Aluminiumsilikat der Alkali- und Erdalkalimetalle bildet. Als Zeolith kann weiterhin ein unter der Bezeichnung ZeoCem ECO 200 von der Firma ZeoCem, a.s., 094 34 Bystré 282, Slowakische Republik vermarktetes Produkt verwendet werden.

Gemäß einer weiteren Ausführungsart werden das Eisenhydroxid enthaltende Produkt und das Zeolith enthaltende Produkt in einem Verhältnis von 95:05 Gewichtsprozent bis 05:95 Gewichtsprozent, vorzugsweise von 80:20 Gewichtsprozent bis 20:80 Gewichtsprozent, verwendet. Nach den bisherigen Untersuchungen ist innerhalb der angegebenen Mengenverhältnisse die Reaktionsgeschwindigkeit im Wesentlichen unabhängig von dem gewählten Mengenverhältnis der beiden Produkte.

Gemäß einer weiteren Ausführungsart wird die Einsatzmenge des Eisenhydroxids stöchiometrisch auf der Grundlage des Volumenstroms des produzierten Biogases und des darin enthaltenen Schwefelwasserstoffs ermittelt und das Eisenhydroxid zusammen mit Zeolith entsprechend zugegeben.

Die Erfindung wird nachfolgend anhand der Ergebnisse einer Untersuchung betreffend die Bestimmung der Reaktionsgeschwindigkeiten des Produkts FerroSorp® DGp in verschiedenen Mischungsverhältnissen mit dem Produkt "Zeolith", bei einer Betriebstemperatur von 40°C näher erläutert. In den anliegenden Figuren gezeigt sind:

Fig. 1    die Lichtdurchlässigkeit-Zeit-Kurven durchgeführter Reaktionen mit Proben 1 bis 16;

Fig. 2    graphische Darstellung der Reaktionszeiten der untersuchten Proben 1 bis 16.

**Datum der Probenahme:** 24.11.2016
**Versuchszeitraum:** 24.11. - 02.12.2016

Ziel der Untersuchung ist es, die Reaktionsgeschwindigkeit von verschiedenen eisenhaltigen Produkten mit Schwefelwasserstoff unter anaeroben Bedingungen zu untersuchen. Dafür wird das FerroSorp® DGp in verschiedenen Mischungsverhältnissen mit dem Produkt "Zeolith", in gleichen Konzentrationen bezogen auf den Eisen (Fe)-Gehalt, untersucht. Durch die gewonnenen Ergebnisse können die verschiedenen auf Eisen basierten Produkte bezüglich ihres Reaktionsstartpunktes und der Reaktionsdauer miteinander verglichen werden. So kann dann festgestellt werden, welche dieser Produkte am besten und schnellsten reagieren und welche für die Entschwefelung eher ungeeignet sind.

**Geräte:**

Analysenwaage *Radwag AS 220/C/2*
Durchlichtmessgerät auf Horizontalschüttler (Eigenbau)
Wärmedämmende, beheizte Kiste (Eigenbau)
PicoLog Datenerfassungsprogramm mit Laptop "Lenovo G700"

**Materialien:**

Verschiedene Eisen basierte Reaktionsmittel [FS DGp, FS DG$\mu$, FS DGo]
Produkt "Zeolith"
Ammoniumsulfat ($(NH_4)_2SO_4$)
Malzextrakt
Abwasser der Kläranlage Waßmannsdorf
Verschließbare PET-Flaschen (500 ml)

**Probenvorbereitung und Durchführung:**

16 durchsichtige PET-Flaschen wurden mit jeweils 0,75 g Malzextrakt (als zusätzliche Kohlenstoffquelle) und 1,5 g Ammoniumsulfat (als zusätzliche Schwefelquelle) und den zu untersuchenden Produkten in unterschiedlichen Konzentrationen, bezogen auf Eisen, gefüllt.

In der nachfolgenden Tabelle (1) sind die Einwaagen der Untersuchungsprodukte gelistet. Bei den Proben 1 bis 6 handelt es sich um reine Untersuchungsprodukte FerroSorp® DG$\mu$, DGo sowie DGp und bei den Proben 9 bis 16 handelt es sich um Mischprodukte aus DGp und "Zeolith". Die Proben 7 und 8 wurden als Kontrollansatz (kein Eisengehalt) hinzugefügt und beinhalten das Produkt "Zeolith". Bei den Proben 9 bis 16 wurden imemr die Einwaagen vom Produkt FerroSorp® DGp verwendet und im Verhältnis zum "Zeolith" gesetzt.

**Tabelle 1**: Einwaage der Untersuchungsprodukte

| ID | Produkt | Fe-Gehaltfeucht [%] | TS [%] | Wassergehalt [%] | Fe-Gehalttrocken [%] | Einwaagetrocken [g] | Einwaagefeucht [g] | Fe-Einwaage [g] 0,01 | Einwaage, IST [g] |
|---|---|---|---|---|---|---|---|---|---|
| 1 | FS DGµ | 32,47 | 89,72 | 10,28 | 36,19 | 0,0276 | 0,0308 | | 0,0307 |
| 2 | FS DGµ | 32,47 | 89,72 | 10,28 | 36,19 | 0,0276 | 0,0308 | | 0,0308 |
| 3 | FS Dgo | 68,78 | 99,60 | 0,40 | 69,06 | 0,0145 | 0,0145 | | 0,0143 |
| 4 | FS Dgo | 68,78 | 99,60 | 0,40 | 69,06 | 0,0145 | 0,0145 | | 0,0143 |
| 5 | FS DGp | 31,48 | 87,70 | 12,30 | 35,90 | 0,0279 | 0,0318 | | 0,0318 |
| 6 | FS DGp | 31,48 | 87,70 | 12,30 | 35,90 | 0,0279 | 0,0318 | | 0,0317 |
| 7 | Zeolith | - | - | - | - | - | 0,0318 (*1) | | 0,0316 |
| 8 | Zeolith | - | - | - | - | - | 0,0318 (*1) | | 0,0323 |
| 9 | DGp-Zeolith (20:80) | - | - | - | - | - | 0,0318+0,1272 (*2) | | 0,0319+0,1276 |
| 10 | DGp-Zeolith (20:80) | - | - | - | - | - | 0,0318+0,1272 (*2) | | 0,0316+0,1271 |
| 11 | DGp-Zeolith (40:60) | - | - | - | - | - | 0,0318+0,0477 (*2) | | 0,0320+0,0477 |
| 12 | DGp-Zeolith (40:60) | - | - | - | - | - | 0,0318+0,0477 (*2) | | 0,0322+0,0482 |
| 13 | DGp-Zeolith (60:40) | - | - | - | - | - | 0,0318+0,0212 (*2) | | 0,0316+0,0215 |
| 14 | DGp-Zeolith (60:40) | - | - | - | - | - | 0,0318+0,0212 (*2) | | 0,0320+0,0212 |
| 15 | DGp-Zeolith (80:20) | - | - | - | - | - | 0,0318+0,0080 (*2) | | 0,0320+0,0078 |
| 16 | DGp-Zeolith (80:20) | - | - | - | - | - | 0,0318+0,0080 (*2) | | 0,0322+0,0078 |

Anschließend wurden die Proben mit jeweils 0,5 Liter Abwasser aus dem Zulauf des Klärwerks Waßmannsdorf möglichst zeitnah aufgefüllt. Die Flaschen wurden luftdicht verschlossen und horizontal in das Durchlichtmessgerät, welches auf dem Schüttler angebracht ist, reingelegt. Die selbst gebaute Kiste wurde über den Schüttler gelegt, sodass er sich vollständig in der Kiste befindet. Bei einer Temperatur von 40 °C und einer Frequenz von 125 min-1 wurden die 16 Proben, über eine Versuchsdauer von 10.200 Minuten (etwa 8 Tage), kontinuierlich geschüttelt.

Durch die Temperaturerhöhung, die durch die wärmedämmende und beheizte Kiste gewährleistet wird, wird die Bildung von H2S begünstigt, da die Bakterien schneller und besser wachsen. Daraus resultiert unter anderem eine Erhöhung der Reaktionsgeschwindigkeit der Umsetzung der Eisen basierten Reduktionsmittel mit dem Schwefelwasserstoff. In der Kiste ist ein Ventilator integriert, welcher die Luft homogen verteilt. Mit dem eingebauten Thermostaten lässt sich außerdem jede beliebige Temperatur bis max. 70°C einstellen.

Aufgrund der typischen Zusammensetzung von kommunalem Abwasser ist davon auszugehen, dass unter diesen Bedingungen innerhalb kürzester Zeit anaerobe Verhältnisse in der Flüssigphase ausgebildet werden. Diese führen zu einer chemischen Reduktion des partikulär vorliegenden dreiwertigen Eisens zu zweiwertigem Eisen (1). Zusätzlich kommt es zur mikrobiologischen Bildung von Sulfid bzw. Schwefelwasserstoff. Treffen Sulfid-Ionen (S2-) mit Eisen-Ionen (Fe2+) zusammen, entsteht in einer spontanen Reaktion (2) schwarz gefärbtes Eisensulfid. Dies führt in Abhängigkeit der Reaktionsgeschwindigkeit der untersuchten Produkte nach unterschiedlichen Zeiten zu einer vollständig schwarz gefärbten Flüssigkeit.

$$(1) \qquad Fe^{3+} + e^- \rightarrow Fe^{2+}$$

$$(2) \qquad Fe^{2+} + S^{2-} \rightarrow FeS \downarrow$$

Dadurch, dass die Proben im Laufe des Versuchszeitraums (aufgrund der Schwarzfärbung) immer Trüber werden, nimmt die aufgenommene Spannung beim Durchlichtmessgerät immer weiter ab, bis den Photowiderstand, ab einem bestimmten Anteil an Eisensulfid in der Probe, gar kein Licht mehr erreicht. Dieser Punkt wird als Ende der jeweiligen Probenreaktionsdauer angenommen. Die Messwertaufnahme erfolgt automatisch alle 15 Minuten und mittels der Formel (3)

$$(3)\ Lichtdurchl\ddot{a}ssigkeit = \frac{V(t)}{V_0} * 100\%$$

$V(t)$ = Spannung zum Zeitpunkt t
$V_0$ = Anfangsspannung

direkt in Lichtdurchlässigkeit umgerechnet. Der Anfangswert ist dabei die maximal erreichte Spannung. Diese fällt für alle Proben aufgrund der unterschiedlichen trübenden Wirkung der Reaktionsmittel und der Inhomogenität des Abwassers verschieden aus. Die Färbung der Proben verändert sich ständig. Um die Reaktionszeiten dennoch ermitteln und vergleichen zu können, wird ein einheitliches Schema verwendet.

Als Anfangszeitpunkt ergibt sich dabei derjenige Wert, für den die Lichtdurchlässigkeit erstmalig seit den letzten 12 Messungen (3 Stunden) einen Abfall von 1 % hat. Als Endzeitpunkt wird der Wert gesetzt, bei dem innerhalb von 15 aufeinander folgenden Messungen (3 Stunden) letztmalig ein Abfall von 0,5 % Lichtdurchlässigkeit gemessen wird. Aus der Differenz des Endzeitpunktes und des Anfangszeitpunktes ergibt sich die Reaktionszeit.

**Ergebnisse:**

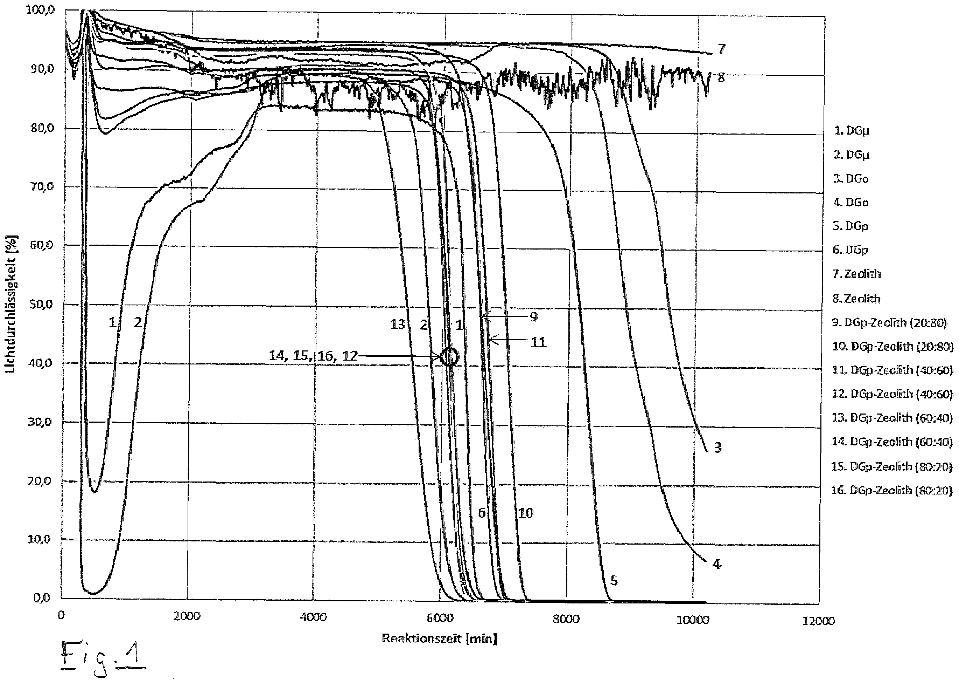

Angestrebt war eine Messdauer von etwa 10 Tagen. Auf Grund eines Software-Updates wurde die Aufzeichnung der Messergebnisse jedoch nach 8 Tagen automatisch abgebrochen. Aus diesem Grund haben die Proben des Produkts DGo im Zeitraum der Datenaufzeichnung nicht zu Ende reagieren können. In Figur 1 wird die Lichtdurchlässigkeit der verschiedenen Proben in Abhängigkeit der Versuchszeit graphisch dargestellt.

Aus den Kurvenverläufen wird ersichtlich, dass die Lichtdurchlässigkeit des Produkts DGµ zu Beginn des Versuchs stark abfällt. Weiterhin fällt auf, dass das reine Produkt "Zeolith" im gesamten Versuchsverlauf nicht reagiert hat.

In Tabelle 2 sind die aus den Messwerten gewonnenen Reaktionszeiten zusammengefasst.

**Tabelle 2:** Reaktionszeiten der untersuchten Proben

| Probe | Reaktionsbeginn [h] | Reaktionsende [h] | Reaktionsdauer [h] |
|---|---|---|---|
| 1. DGµ | 93,5 | 111,25 | 15,25 |
| 2. DGµ | 84 | 105,50 | 21,50 |
| 3. DGo | 142,25 | Kein Ende | - |
| 4. DGo | 133,25 | Kein Ende | - |
| 5. DGp | 117,25 | 146,67 | 29,42 |
| 6. DGp | 101,25 | 116,00 | 14,75 |
| 7. Zeolith | Kein Anfang | Kein Ende | - |
| 8. Zeolith | Kein Anfang | Kein Ende | - |
| 9. DGp-Zeolith (20:80) | 103 | 116,75 | 13,75 |
| 10. DGp-Zeolith (20:80) | 107,5 | 122,75 | 15,25 |
| 11. DGp-Zeolith (40:60) | 100 | 117,75 | 17,75 |
| 12. DGp-Zeolith (40:60) | 93,5 | 110,00 | 16,50 |
| 13. DGp-Zeolith (60:40) | 79,75 | 102,50 | 22,75 |
| 14. DGp-Zeolith (60:40) | 93 | 108,50 | 15,50 |
| 15. DGp-Zeolith (80:20) | 93,25 | 109,00 | 15,75 |
| 16. DGp-Zeolith (80:20) | 94 | 107,00 | 13,00 |

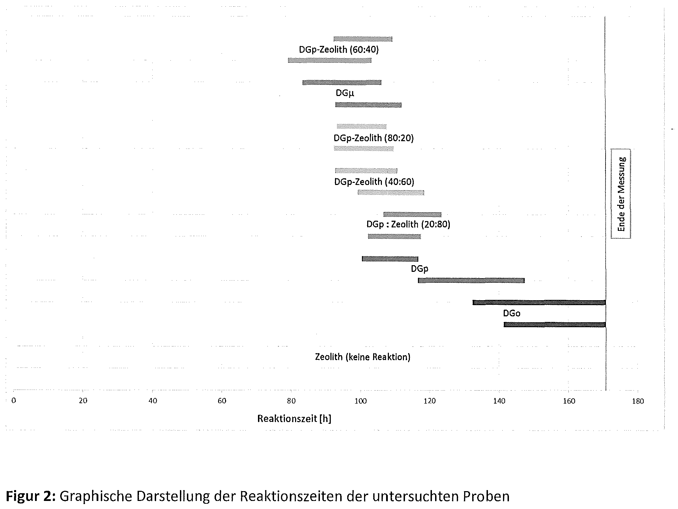

Zur Veranschaulichung sind die Daten der Tabelle 2 in Figur 2 graphisch gegenübergestellt.

Die Doppelbestimmung der untersuchten Proben ist für einen untersuchten Zeitraum von etwa 8 Tage hinreichend genau. Es fällt jedoch auf, dass die Reaktionsstartpunkte der DGp Proben ziemlich weit auseinander liegen.

In der Tabelle 2 und in Abbildung 2 ist zu erkennen, dass die Reaktion der Mischung FerroSorp® DGp - Zeolith (60:40) am schnellsten einsetzt. Es folgt die Probe FerroSorp® DGµ, dicht gefolgt von der Mischung FerroSorp® DGp - Zeolith (80:20). Als nächstes kommt dann das Mischprodukt FerroSorp® DGp - Zeolith (40:60). Der weitere Trend (Reaktionsbeginn) ist in Abbildung 2 gut dargestellt.

Werden die insgesamt für die Umsetzung benötigten Zeiten verglichen, so stellen hier die Mischprodukte FerroSorp® DGp - Zeolith (80:20) und FerroSorp® DGp - Zeolith (20:80) die schnellsten Produktklassen dar. Um nun festzustellen welches dieser beiden Produkte die kürzeste Reaktionsdauer hat, werden die Mittelwerte berechnet.

- Mittelwert Reaktionsdauer Mischprodukt FerroSorp® DGp - Zeolith (80:20) = 14,3 h
- Mittelwert Reaktionsdauer Mischprodukt FerroSorp® DGp - Zeolith (20:80) = 14,5 h

Der direkte Vergleich der gesamten Reaktionszeiten der beiden Mischprodukte liefert die Erkenntnis, dass sie trotz umgekehrter Mischungsverhältnisse etwa gleich schnell sind.

Im Gegensatz zu den Mischprodukten weisen die Produkte FerroSorp® DGµ und FerroSorp® DGp längere Umsetzungszeiten auf.

- Mittelwert Reaktionsdauer Mischprodukt FerroSorp® DGµ = 19,6 h
- Mittelwert Reaktionsdauer Mischprodukt FerroSorp® DGp = 22,1 h

**Zusammenfassung:**

Bei der Untersuchung der zeitlichen Umsetzung von Schwefelwasserstoff mit den Eisen basierten Reduktionsmitteln wurde festgestellt, dass das Mischprodukt FerroSorp® DGp -Zeolith (60:40) und das Produkt DGµ am schnellsten einsetzen. Entgegen den Erwartungen setzte der Reaktionsbeginn der Probe FerroSorp® DGp in einer hohen zeitlichen Abweichung voneinander ein.

Die kürzeste Gesamtdauer der Reaktion konnte durch die Mischproben erreicht werden, wobei die Mischungen FerroSorp® DGp - Zeolith (80:20) sowie FerroSorp® DGp - Zeolith (20:80) am schnellsten umgesetzt wurden.

Das Produkt FerroSorp® DGµ ist besonders feingemahlen und kommt aus wirtschaftlichen Gründen für den Einsatz in größeren Mengen zwecks Ausfällung des Schwefeldioxids bei der Biogaserzeugung nicht in Betracht.

Nachfolgend sind die Produktspezifikationen der Eisenhydroxid enthaltenden Produkte FerroSorp® DGp und FerroSorp® DGp+K und und FerroSorp® DGμ angegeben, die für die vorstehende Vergleichsuntersuchung verwendet wurden. Die Produkte Ferrosorp® DGp und FerroSorp® DGp+K werden vorzugsweise für die Anwendung der Erfindung verwendet.

Produktspezifikation

FerroSorp® DGp

| Stoffgruppe | : | Eisen(III)-oxidhydrat, Eisenoxihydroxid |
|---|---|---|
| Formel | : | FeO(OH) |
| Eisengehalt ($Fe^{3+}$) | : | mind. 33 %, bez. auf den Trockenstoff |
| Trockenstoffgehalt | : | mind. 85 % |
| Mangangehalt | : | ca. 1,2 %, bez. auf den Trockenstoff |
| Farbe | : | rotbraun |
| Beschaffenheit | : | pulverförmig |
| Schüttdichte | : | 600 ± 50 g/l (lockere Schüttung), bei ca. 85 - 90 % TS |
| | | 680 ± 50 g/l (verdichtete Schüttung), bei ca. 85 - 90 % TS |
| Kornbereich | : | 0 - 0,5 mm |

| Schwermetallgehalte Schwermetall | chem. Symbol | Gehalt [mg/kg TS] | | typische Werte [mg/kg TS] | | Grenzwerte gem. DüMV [mg/kg TM] |
|---|---|---|---|---|---|---|
| Arsen | As | < | 80 | | 20 - 70 | 80[1] |
| Blei | Pb | < | 50 | < | 40 | 150 |
| Cadmium | Cd | < | 1,0 | | 0,1 - 0,6 | 1,0 |
| Chrom ges. | Cr | < | 20 | < | 20 | n.a. (Kennz. ab 300) |
| Chrom (VI) | Cr | < | 1,0 | | 0,1 - 0,5 | 2,0 |
| Kupfer | Cu | < | 20 | | 2 - 15 | - |
| Nickel | Ni | < | 100 | | 30 - 95 | 120[1] |
| Quecksilber | Hg | < | 0,4 | | 0,05 - 0,3 | 1,0 |
| Zink | Zn | < | 500 | | 100 - 450 | - |
| Thallium | Tl | < | 0,4 | < | 0,4 | 1,0 |
| Perfluorierte Tenside | PFT | < | 0,02 | < | 0,02 | 0,1 |

Produktspezifikation

FerroSorp® DGp+K

| Stoffgruppe | : | Eisen(III)-oxidhydrat |
|---|---|---|
| Formel | : | FeO(OH) |
| Eisengehalt ($Fe^{3+}$) | : | mind. 34 %, bez. auf den Trockenstoff |
| Trockenstoffgehalt | : | mind. 85 % |
| Farbe | : | rotbraun |
| Beschaffenheit | : | pulverförmig |
| Schüttdichte | : | 630 ± 60 g/l (lockere Schüttung), bei ca. 85 - 90 % TS |
| | | 730 ± 90 g/l (verdichtete Schüttung), bei ca. 85 - 90 % TS |
| Kornbereich | : | 0 - 0,5 mm |

| Schwermetallgehalte Schwermetall | chem. Symbol | Gehalt [mg/kg TS] | | typische Werte[2) [mg/kg TS] | | Grenzwerte gem. DüMV [mg/kg TM] |
|---|---|---|---|---|---|---|
| Arsen | As | | < 80 | | 20 - 70 | 80[1) |
| Blei | Pb | < | 50 | < | 40 | 150 |
| Cadmium | Cd | < | 1,0 | | 0,1 - 0,6 | 1,0 |
| Chrom ges. | Cr | < | 20 | < | 30 | n.a. (Kennz. ab 300) |
| Chrom (VI) | Cr | < | 1,0 | < | 1,0 | 2,0 |
| Kupfer | Cu | < | 20 | < | 5 - 20 | - |
| Nickel | Ni | < | 100 | | 30 - 95 | 120[1) |
| Quecksilber | Hg | < | 0,4 | | 0,05 - 0,3 | 1,0 |
| Zink | Zn | < | 500 | | 100 - 450 | - |
| Thallium | Tl | < | 0,4 | < | 0,4 | 1,0 |

Produktspezifikation

FerroSorp® DGμ

| Stoffgruppe | : | Eisen(III)-oxidhydrat, Eisenoxihydroxid | |
|---|---|---|---|
| Formel | : | $FeO(OH)$ | |
| Eisengehalt ($Fe^{3+}$) | : | mind. 37 %, typisch 38 - 39 % bez. auf den Trockenstoff | |
| Restfeuchte | : | max. 15 %, typisch 10 % | |
| Mangangehalt | : | ca. 1,2 %, bez. auf den Trockenstoff | |
| Farbe | : | rotbraun | |
| Beschaffenheit | : | pulverförmig | |
| Schüttdichte | : | 450 $\pm$ 50 g/l | |
| Kornbereich | : | 0 - 0,05 mm | |
| typische Kornanteile | : | < 0,001 mm | ca. 15 - 25 % |
| | | 0,001 - 0,01 mm | ca. 50 - 60 % |
| | | 0,01 - 0,05 mm | ca. 15 - 25 % |
| | | 0,250 - 0,355 mm | ca. 15 - 20 % |
| | | > 0,5 mm | < 5 % |

| Schwermetallgehalte Schwermetall | chem. Symbol | Gehalt [mg/kg TS] | | typische Werte [mg/kg TS] | Grenzwerte gem. DüMV [mg/kg TM] |
|---|---|---|---|---|---|
| Arsen | As | < | 80 | 40 - 70 | 80 |
| Blei | Pb | < | 50 | < 30 | 150 |
| Cadmium | Cd | < | 1,5 | 0,1 - 0,8 | 1,5 |
| Chrom ges. | Cr | < | 10 | < 10 | n. a. (Kennz. ab 300) |
| Chrom (VI) | Cr | < | 1,0 | < 1,0 | 2,0 |
| Kupfer | Cu | < | 10 | 2 - 4 | - |
| Nickel | Ni | < | 100 | 25 - 70 | 120 |
| Quecksilber | Hg | < | 0,2 | 0,05 - 0,1 | 1,0 |
| Zink | Zn | < | 250 | 100 - 200 | - |
| Thallium | Tl | < | 0,4 | < 0,4 | 1,0 |
| Perfluorierte Tenside | PFT | < | 0,02 | < 0,02 | 0,1 |

Die Einsatzmenge des Eisenhydroxids kann stöchiometrisch auf der Grundlage des Volumenstroms des produzierten Biogases und des darin enthaltenen Schwefelwasserstoffs anhand des nachfolgenden Schemas ermittelt werden. Bei dem Berechnungsbeispiel wurden eine Biogasproduktion von 144,25 m³/h und ein $H_2S$-Gehalt im Rohgas von 1.000 ppm angenommen. Um den $H_2S$-Gehalt auf 0 ppm zu reduzieren, wäre der Einsatz von 1 kg/h FerroSorp® DG erforderlich.

Eine Biogasproduktion von 50 m³/h entspricht etwa 100 kW elektrischer Leistung. Im Mittel weisen Biogasanlagen in Deutschland eine elektrische Leistung von 500 kW auf. Der Zeolith kann in einem geeigneten Mengenverhältnis mit dem Eisenhydroxid zugegeben werden, insbesondere in einem Bereich gemäß Anspruch 8.

**Kalkulation der Einsatzmengen von FerroSorp® DG**

**Eingangsparameter**

| | | |
|---|---|---|
| Gasproduktion: | 144,250 | m³/h |
| $H_2S$-Gehalt im Rohgas: | 1.000,000 | ppm |
| Zielgröße $H_2S$-Gehalt im Reingas: | 0,000 | ppm |
| $H_2S$-Reduktion: | 1.000,000 | ppm |
| ß-Faktor: | 1,000 | (Annahme) |

**Interne Parameter**

| | |
|---|---|
| Molmasse Fe: | 55,847 g/mol |
| Molmasse $H_2S$: | 34,080 g/mol |
| Molmasse S: | 32,064 g/mol |
| Gaskonstante: | 22,400 l/mol |
| Eisengehalt im FerroSorp® DG: | 360,000 g/kg |

**Reaktionen**

$$Fe^{3+} + e^- ---> Fe^{2+}$$

$$Fe^{2+} + S^{2-} ---> FeS$$

**Berechnung**

| | | | |
|---|---|---|---|
| $H_2S$-Abreicherung: | 0,045 | mol/m³ | bzw. |
| | 1,521 | g/m³ | |
| S-Abreicherung: | 1,431 | g/m³ | |
| S-Abreicherungsmenge: | 206,484 | g/h | bzw. |
| | 6,440 | mol/h | |
| Fe-Bedarf: | 360 | g/h | |
| **FerroSorp® DG - Bedarf:** | **1,00** | **kg/h** | **bzw.** |

(fortgesetzt)

| 23,976 | kg/d | bzw. |
|---|---|---|
| 8,751 | t/a | |

**Patentansprüche**

1. Verwendung von Eisenhydroxid bei der Biogaserzeugung als Schwefelwasserstoff ausfällender Zusatz zur Biomasse, **dadurch gekennzeichnet, dass** zusätzlich Zeolith als die Reaktionsgeschwindigkeit der Ausfällung des Schwefelwasserstoffs durch Eisenhydroxid erhöhender Zusatz zur Biomasse verwendet wird.

2. Verwendung nach Anspruch 1, bei der das Eisenhydroxid und der Zeolith vorgemischt und der Biomasse während der Biogaserzeugung zugeführt werden.

3. Verwendung nach Anspruch 1, bei der das Eisenhydroxid und der Zeolith der Biomasse während der Biogaserzeugung gesondert zugeführt werden.

4. Verwendung nach Anspruch 1, bei der das Eisenhydroxid und der Zeolith mit der zu fermentierenden Biomasse vorgemischt und die Vormischung dem Gärprozess zugeführt wird.

5. Verwendung nach einem der Ansprüche 1 bis 4, bei der das Eisenhydroxid und der Zeolith mittels Feststoffschnecken der Biomasse in einem Biogasreaktor zugeführt werden.

6. Verwendung nach einem der Ansprüche 1 bis 5, bei der ein pulverförmiges Reaktionsmittel auf der Basis von Eisenhydroxid mit einem Eisengehalt von mindestens 10 %, vorzugsweise von mindestens 20 %, vorzugsweise von mindestens 33 %, bezogen auf den Trockenstoff und einem Trockenstoffgehalt von mindestens 50 %, vorzugsweise von mindestens 70 %, vorzugsweise von mindestens 85 % verwendet wird.

7. Verwendung nach einem der Ansprüche 1 bis 6, bei dem als Zeolith ein aufbereitetes Naturgestein verwendet wird, dessen wesentlicher Bestandteil hydratisiertes Aluminiumsilikat der Alkali- und Erdalkalimetalle bildet.

8. Verwendung nach einem der Ansprüche 1 bis 7, bei dem das Eisenhydroxid enthaltende Produkt und das Zeolith enthaltende Produkt in einem Verhältnis von 95:5 Gewichtsanteilen bis 5:95 Gewichtsanteilen verwendet werden, vorzugsweise von 80:20 Gewichtsanteilen bis 20:80 Gewichtsanteilen.

9. Verwendung nach einem der Ansprüche 1 bis 8, bei der die Einsatzmenge des Eisenhydroxids stöchiometrisch auf der Grundlage des Volumenstroms des produzierten Biogases und des darin enthaltenen Schwefelwasserstoffs ermittelt und das Eisenhydroxid zusammen mit dem Zeolith entsprechend zugegeben wird.

**Claims**

1. A use of iron hydroxide in biogas production as a hydrogen-sulfide-precipitating additive for biomass, **characterized in that** zeolite is also used as the additive to the biomass to increase the reaction speed of the precipitation of the hydrogen sulfide by iron hydroxide.

2. The use according to claim 1, wherein the iron hydroxide and the zeolite are premixed and fed to the biomass during biogas production.

3. The use according to claim 1, wherein the iron hydroxide and the zeolite are fed to the biomass separately during biogas production.

4. The use according to claim 1, wherein the iron hydroxide and the zeolite are premixed with the biomass to be fermented, and the pre-mixture is fed to the fermentation process.

5. The use according to one of claims 1 to 4, wherein the iron hydroxide and the zeolite are fed to the biomass in a biogas reactor by solid screw conveyors.

**6.** The use according to one of claims 1 to 5, wherein a powdered reactant based on iron hydroxide is used with an iron content of at least 10%, preferably at least 20%, and preferably at least 33% relative to the dry material, and with a dry material content of at least 50%, preferably at least 70%, and preferably at least 85%.

**7.** The use according to one of claims 1 to 6, wherein a prepared natural rock is used as the zeolite whose fundamental component forms a hydrated aluminum silicate of alkaline and earth alkaline metals.

**8.** The use according to one of claims 1 to 7, wherein the iron-hydroxide-containing product and the zeolite-containing product are used at a ratio of 95:5 parts by weight to 5:95 parts by weight, preferably from 80:20 parts by weight to 20:80 parts by weight.

**9.** The use according to one of claims 1 to 8, wherein the used amount of iron hydroxide is ascertained stoichiometrically on the basis of the volumetric flow of the produced biogas and the hydrogen sulfide contained therein, and the iron hydroxide is correspondingly added together with the zeolite.

**Revendications**

**1.** Utilisation d'hydroxyde ferrique lors de la production de biogaz en tant qu'additif précipitant d'hydrogène sulfuré à la biomasse, **caractérisée en ce que** de la zéolithe est en outre utilisée en tant qu'additif à la biomasse pour accroître la vitesse de réaction de la précipitation d'hydrogène sulfuré par l'hydroxyde ferrique.

**2.** Utilisation selon la revendication 1, dans laquelle l'hydroxyde ferrique et la zéolithe sont préalablement mélangés et fournis à la biomasse pendant la production de biogaz.

**3.** Utilisation selon la revendication 1, dans laquelle l'hydroxyde ferrique et la zéolithe sont fournis séparément à la biomasse pendant la production de biogaz.

**4.** Utilisation selon la revendication 1, dans laquelle l'hydroxyde ferrique et la zéolithe sont préalablement mélangés avec la biomasse à faire fermenter et le prémélange est fourni au processus de fermentation.

**5.** Utilisation selon l'une des revendications 1 à 4, dans laquelle l'hydroxyde ferrique et la zéolithe sont fournis à la biomasse dans un réacteur de biogaz à l'aide de vis sans fin pour matières solides.

**6.** Utilisation selon l'une des revendications 1 à 5, dans laquelle un réactif pulvérulent à base d'hydroxyde ferrique avec une teneur en fer d'au moins 10%, de préférence d'au moins 20%, de préférence d'au moins 33%, par rapport à la matière sèche et une teneur en matière sèche d'au moins 50%, de préférence d'au moins 70%, de préférence d'au moins 85% est utilisé.

**7.** Utilisation selon l'une des revendications 1 à 6, dans laquelle une pierre naturelle traitée, dont le composant principal forme du silicate d'aluminium hydraté des métaux alcalins et alcalino-terreux, est utilisée en tant que zéolithe.

**8.** Utilisation selon l'une des revendications 1 à 7, dans laquelle le produit contenant de l'hydroxyde ferrique et le produit contenant de la zéolithe sont utilisés selon un rapport de 95:5 parties en poids à 5:95 parties en poids, de préférence de 80:20 parties en poids à 20:80 parties en poids.

**9.** Utilisation selon l'une des revendications 1 à 8, dans laquelle la quantité d'hydroxyde ferrique utilisée est déterminée stoechiométriquement sur la base du flux volumique du biogaz produit et de l'hydrogène sulfuré contenu dans celui-ci, et l'hydroxyde ferrique est ajouté de façon correspondante ensemble avec la zéolithe.

Lichtdurchlässigkeit [%]

100,0

90,0

80,0

70,0

60,0

50,0

40,0

30,0

20,0

10,0

0,0

0    2000    4000    6000    8000    10000    12000

Reaktionszeit [min]

1. DGμ
2. DGμ
3. DGo
4. DGo
5. DGp
6. DGp
7. Zeolith
8. Zeolith
9. DGp-Zeolith (20:80)
10. DGp-Zeolith (20:80)
11. DGp-Zeolith (40:60)
12. DGp-Zeolith (40:60)
13. DGp-Zeolith (60:40)
14. DGp-Zeolith (60:40)
15. DGp-Zeolith (80:20)
16. DGp-Zeolith (80:20)

14, 15, 16, 12

7
8
9
11
13  2  1
6  10
1  2
3
4
5

Fig. 1

**Figur 2:** Graphische Darstellung der Reaktionszeiten der untersuchten Proben

EP 3 438 268 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 60014995 A
- EP 1577269 B2
- DE 102007003532 A1
- JP 2001316302 A
- CN 105617996 A
- KR 20080045818 A
- EP 298386 A2

**In der Beschreibung aufgeführte Nicht-Patentliteratur**

- **SAHM.** Biologie der Methanbildung. *Chem.-Ing. Tech.,* 1981, vol. 53 (11), 854-863
- **DR. SABINE RAHN ; DETLEF GÜßBACHER ; EVGENIJ MARCENKO.** Eisenhydroxid ist nicht gleich Eisenhydroxid. *Joule 5.20154,* 58-60